# EUROPEAN PATENT APPLICATION

(11) **EP 2 246 436 A1**
(43) Date of publication of application: **03.11.2010**
(21) Application number: 09425167.5
(22) Date of filing: 30.04.2009
(51) Int. Cl.: C12P 3/00, C12P 5/00

(54) **Semi-continuous process for hydrogen production from organic waste**

(71) Applicant: UNIVERSITA DEGLI STUDI DI CAGLIARI, 90124 Cagliari CA (IT)
(72) Inventor: Muntoni, Aldo, 09126 Cagliari (IT); De Gioannis, Giorgia, 09126 Cagliari (IT)
(74) Representative: Palladino, Saverio Massimo

(57) **Abstract**

There is described a process for semi-continuous hydrogen production from mixtures of organic waste, which can advantageously be integrated in a single process for the production of hydrogen, methane and compost, of which it constitutes the first phase; the invention also relates to a plant for implementation of the process.

## Description

### Field of the invention

The present invention relates to a process for semi-continuous hydrogen production from mixtures of organic waste, which can advantageously be integrated in a single process for the production of hydrogen, methane and compost, of which it constitutes the first biological phase; the invention also relates to a plant for implementation of the process.

### Background art

Biological treatments of biodegradable organic waste, advisable and by now necessary alternatives to mere direct disposal, are the subject of great interest and numerous research studies as they offer the advantage of allowing recycling and/or energy production, reducing the environmental impact of said waste and improving the system energy balance by allowing the entry of renewable energy. Organic wastes that can be treated with these processes comprise, among others, putrescible fractions of urban waste from separate waste collection or from mechanical sorting, solid or liquid waste from livestock processes (breeding and slaughter of pigs, production of cheeses, etc.) or agro-industrial processes (production of oil, wine, sugar, etc.), waste from urban sewage treatment.

Among the various processes that can be used to treat biodegradable organic waste, it is necessary to distinguish between aerobic and anoxic and anaerobic processes. The aerobic metabolism is typical of microorganisms that require molecular oxygen, the anoxic metabolism requires oxygen in bound form (e.g. nitrates or sulphates), the anaerobic metabolism requires the total absence of oxygen. Anaerobic processes, although characterised by slower degradation of the organic substrate with respect to aerobic processes, allow the production of gases with high energy content (methane, hydrogen) to be coupled with degradation/stabilisation of the substrate.

The first studies and the first applications in this field related to the application of aerobic processes for the production of compost to be used as soil ameliorant. With regard to processes in the absence of molecular oxygen, anaerobic digestion has been developed, aimed both at stabilising the biodegradable organic substance and at producing methane.

Recently, in the light of impacts related to combustion processes, and in particular with the worsening of those caused by the emission of greenhouse gases into the atmosphere, such as carbon dioxide (also produced by combustion for energy purposes of methane, itself a powerful greenhouse gas), interest in the hydrogen gas production has developed. The energy option represented by hydrogen is one of the most attractive for the future, due to its noteworthy specific energy content and the high environmental compatibility of use. Free hydrogen can be produced through electrolysis of water or thermochemical and radiolytic processes. In spite of the environmentally compatible use of hydrogen, the majority of the aforesaid processes are based on the use of non-renewable energy sources, such as natural gas, hydrocarbons and coal, and are, moreover, often energy-consuming and costly. In this context, biological production appears interesting, above all in view of desirable delocalisation of part of the energy production to a network of micro-production centres spread over the territory. Different types of bacteria, including anaerobic, aerobic, photosynthetic and cyanobacteria, are capable of producing hydrogen from degradable biomasses. The interesting prospects offered by biological hydrogen production have given rise, among other things, to the hypothesis of growing vegetable species specifically to be used as substrate. Nonetheless, the need to grow biomass for this purpose appears debatable in the light of the fact that noteworthy quantities of biodegradable organic substance are already present in waste of various types and, moreover, these represent an environmental problem that requires to be managed appropriately. Therefore, it is believed that the hydrogen production from waste streams represents one of the most promising options.

Among the various potentially usable biological processes, anaerobic fermentation has numerous advantages. In fact, the bacterial populations involved are "flexible" in terms of type of degradable substrate and, moreover, the process does not require the presence of light, and can therefore be conducted in relatively small reactors and during the day or at night without distinction. In the light of these considerations, anaerobic digestion appears an extremely interesting option for biological hydrogen production.

The problems which currently hinder the development of biological hydrogen production lie partly in the general characteristics of anaerobic digestion processes, even if targeted only at conventional methane production, and partly in the peculiarities inherent to fermentative hydrogen production. In fact, processes for anaerobic bio-treatment of waste, especially if compared to aerobic processes, are generally characterised by an intrinsic vulnerability due to process development according to phases that follow one another in series (therefore, if a single phase is blocked this blocks the entire process) and to the fact that the microorganisms involved are highly sensitive to the action of numerous inhibiting elements, both exogenous, i.e. introduced into the system with the waste, and endogenous, such as some of the products of biochemical reactions. The build-up of inhibiting substances can also be accentuated by the relatively common practice of recirculation of process waters and sludge. Besides, it must be considered that the various types of microorganisms operate with different efficiencies on wastes of different nature.

Added to these possible negative trends of the anaerobic biochemical system in general are distinctive problems, if the objective is not conventional methane production, but hydrogen production. In the first place it must be considered that the natural process tends to evolve by converting, spontaneously and as rapidly as possible, the hydrogen produced into methane. Therefore, if significant quantities of hydrogen are to be produced in a stable manner, it is necessary to achieve and maintain particular conditions such as to maximise hydrogen production and simultaneously inhibit the natural activity of consumption thereof by methanogenic bacteria. In particular, the pH value of the system and the retention time are among the elements that seem to play a key role; in particular, the retention time must be such as to limit the development of methanogenic bacteria and encourage rapid removal of any that do manage to develop, while also ensuring adequate time for the action of the hydrolytic and fermentative bacteria that produce gaseous hydrogen. The importance of identifying these conditions, and the methods and systems which allow them to be achieved and maintained in the simplest and most cost-effective manner possible, is therefore evident.

The article "Hydrogen production through anaerobic digestion of different solid and liquid waste: batch and semi-continuous tests", G. De Gioannis et al, Proc. Sardinia 2007, Eleventh International Waste Management and Landfill Symposium, 1-5 October 2007 (published on CD-ROM) describes a process for biological hydrogen production from different mixtures of urban waste, agroalimentary waste and waste from the livestock industry, and hypothesises, in the conclusions, introduction of such process into a three-phase integrated system in which hydrogen is produced in the first phase, methane in the second and compost in the third one.

To summarise the above, there is still the need in the sector to identify an anaerobic biological process capable of producing high quantities of hydrogen, with essentially constant production of the element in time, which can be integrated with other processes for biochemical transformation of organic waste so as to provide a process whose end products are all useful and re-usable.

### Summary of the invention

These objects are accomplished with the present invention, which in a first aspect relates to a process for biological production of hydrogen through semi-continuous anaerobic digestion at a temperature between 30 and 40 °C of a mixture of waste having a total solids content not exceeding 10% in weight; the mixture is constituted by biodegradable organic fractions of urban waste, both from separate refuse collection and from mechanical sorting, from vegetation waters deriving from olive oil production and from active sludge from urban sewage purification; the process is characterised by being run with an average residence time of the mixture in the hydrogen production reactor between two to four days, obtained through daily removal of a portion corresponding to approximately 1/n of the volume of mixture in the reactor, where n indicates the number of days of desired average residence time, and feeding an equivalent volume of fresh analogous mixture into the reactor.

The inventors have found in general that by operating on mixtures of the aforesaid components appreciable quantities of hydrogen are produced and that by appropriately selecting the weight ratios between components and, above all, optimising the retention time value, the system remains in biochemical equilibrium, i.e. without giving rise to reaction trends, for an indefinite time, during which hydrogen is produced in large and essentially constant quantities. The optimal time was found to be between two and four days, and preferably two days. Moreover, the supply of active sludge from urban sewage purification to the reaction mixture makes it possible to avoid recirculation of process water in the system, normally adopted in known anaerobic digestion systems, which in time causes accumulation in the reactor of toxic substances that inhibit the action of microorganisms. In fact, the use of active sludge makes it possible to achieve the water content required for a wet process while simultaneously supplying the system with microorganisms particularly active in hydrolysis of the substrate and, being facultative, with low sensitivity to any modest accidental oxygen intrusion. Moreover, with the process of the invention it is not necessary to perform a thermal or chemical pretreatment of the biomass targeted at inhibiting methanogenic microorganisms and selecting hydrogen producing microorganisms, a practice used in some prior art systems.

### Brief description of the figures

The invention will be described below with reference to the figures, wherein:
- Fig. 1 schematically represents a possible integrated plant for the production of hydrogen, methane and compost;
- Fig. 2 shows the hydrogen production trend during a semi-continuous test conducted by the inventors;
- Fig. 3 shows the methane production trend during a semi-continuous test conducted by the inventors.

### Detailed description of the invention

The mixture of biodegradable organic waste usable to produce hydrogen according to the invention is composed, as stated previously, by putrescible organic fractions of urban waste, both from separate waste collection and from mechanical sorting, vegetation waters from olive oil production and active sludge from urban sewage purification; this waste will be referred to hereafter with the following abbreviations:
- vegetation waters from olive oil production = OMW
- active sludge from urban sewage purification = AS
- putrescible organic fractions of urban waste = MSWOF

The various components can be present in the mixture in the following percentages in weight:
- OMW between 5 and 25%, preferably 18-22%;
- AS between 45 and 70%, preferably 50-60%;
- MSWOF between 18 and 35%, preferably 22-30%.

The mixture composed of 20% OMW + 55% AS + 25% MSWOF is particularly preferred: in fact, the inventors have observed that this particular mixture allows the reaction to be sustained without requiring to add specific additives, at the beginning of or during the process, to regulate the pH of the reaction mixture to optimal values between approximately 5 and 5.5, a practice which is instead essential with prior art techniques. Moreover, if the optimal retention time of two days is adopted, this mixture is the one which achieves maximum hydrogen production values per unit of mass of volatile solids fed and removed during the process.

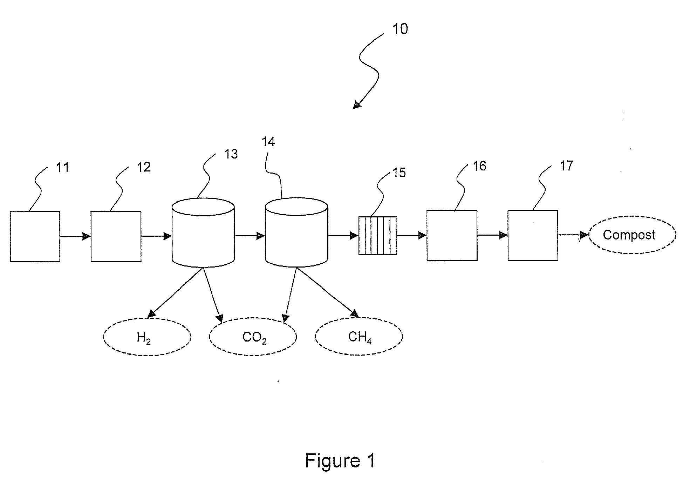

Before being fed to the hydrogen production reactor, the biodegradable fractions of urban waste are subjected to an operation of size reduction (in a suitable station equipped with cutting tools), in which the solid components are reduced to dimensions of less than 50 mm, to favour homogeneity of the reaction system and subsequent pumping of the mixture, as well as to speed up reactions. The MSWOF fraction thus pretreated is fed to a heated mixer together with the liquid or fluid fractions of the mixture. Fig. 1 schematically shows a possible plant, 10, for waste treatment according to the invention, suitable to operate according to this operating method; in the figure, the products extracted from the various reactors or elements of the plant are indicated in the dashed ovals.

Element 11 represents the size reducing station and element 12 represents the mixing station.

The mixture thus prepared is then pumped to a reactor, element 13 in Fig. 1, of the continuous stirred-tank chemical or bio-chemical reactor type (known in the sector as CSTR), equipped with instruments to control the main operating parameters (pH, temperature) and with a thermostatic system to maintain the temperature of the mass at values between about 30 and 40 °C; in fact, the inventors have observed that the hydrogen producing reaction proceeds with high yields already when the reaction mixture temperature is maintained in this interval, preferably between 35 and 40 °C, and even more preferably at a temperature of about 39 °C, without the need to adopt thermophilic conditions (of about 55 °C, adopted in other analogous known processes). Both the feed system and the reactor are gas tight, to allow anaerobic conditions to be established in the latter (preventing air from entering from the outside); the reactor is also provided with gas-tight ports to take samples of the reaction mixture for checking the reaction trend with time. The gas produced is conveyed to a gasometer; ports are positioned upstream of the gasometer, along the gas conveying duct, and downstream thereof, for sampling and analysing the gas. This type of reactor is known in the sector of biochemical plants and requires no further description herein.

In this reactor, the mixture is undergoes hydrolysis and acid fermentation during which it is transformed essentially into volatile acids, hydrogen and carbon dioxide by hydrolysing and fermenting bacteria.

The reaction mixture is fed to the reactor according to a time schedule such that each day a volume of approximately half the volume of reaction mixture present in the reactor is discharged and an equivalent volume of fresh mixture is fed. By operating according to this schedule, an average residence time of the reaction mixture in the reactor for hydrogen production of 2 days is determined, found to be optimal in terms of maximum hydrogen production per unit of mass of volatile solids fed and removed. As already stated, the inventors have found that adoption of a suitable retention time, between two and four days, is a fundamental condition for obtaining the desired characteristics of quantity and stability in time of hydrogen production. In fact, by adopting a retention time of 2 days, essentially constant average productions, about 70 NI (normal litres, i.e. gas volume in litres measured at ambient pressure and T = 25 °C) of hydrogen gas per day are obtained per kg of volatile solids fed to the reactor; by operating with a residence time extended up to 4 days (i.e. with daily extraction and feed of a reaction mixture volume of a fourth of the volume of the reactor) hydrogen production decreases to values in the order of 50 NI of hydrogen per kg of volatile solids fed to the reactor, while by operating with a residence time of more than 4 days the reduction in hydrogen production becomes progressively more accentuated and the system, intended as hydrogen production, tends to block or to evolve towards methanogenic conditions, therefore unsuitable for significant and stable hydrogen production.

The reduction of volatile solids achievable, an indicator of waste stabilisation, is stably above 30% in weight, on average approximately 32%. The gas produced stably contains 35-45% in volume of hydrogen, the rest of the gas being constituted essentially of carbon dioxide.

As stated previously, the process and the reactor for hydrogen production are preferably combined with further biological process phases and reactors or equipment for methane and compost production, to provide an integrated process and an integrated plant for producing the three products. In detail, these two further biological stages follow that of hydrogen production, to provide an integrated process and a plant in which the first two anaerobic biological stages (hydrogen and methane production) are destined for the production of renewable energy, and the third aerobic stage for the production of a material to be reused in agriculture if the putrescible fractions of urban waste come from separate refuse collection, or for environmental restoration if the putrescible fractions of urban waste come from mechanical sorting.

In fact, the inventors have found that the product extracted from the hydrogen production bioreactor has optimal characteristics to be fed to a bioreactor, still anaerobic, for methane production; in fact, it contains both acetic acid, the main substrate for methane production, and other volatile organic acids easily convertible, again in the second biological stage, into acetic acid and therefore ultimately into methane. The inventors were able to verify that the methanogenic phase applied to the product extracted from the hydrogen production bioreactor leads to an average methane production of approximately 700 NI/kg SV (volatile solids fed to the methanogenic reactor).

Fig. 1 schematically shows this configuration, in which the bioreactor for methane production, 14, is immediately downstream of the bioreactor 13 for hydrogen production. The volume of reaction mixture extracted daily from the hydrogen production bioreactor 13 is fed directly to the bioreactor 14 containing acetophilic methanogenic bacteria that directly produce methane and carbon dioxide and acetogenic bacteria that convert the other volatile acids into acetic acid, which in turn is converted by the former into methane and carbon dioxide.

The methane production stage requires an inoculum of methanogenic biomass in the initial phase; during normal operation a temperature between 35 and 40 °C is adopted, i.e. the same as the hydrogen production phase, but unlike the latter methane production requires residence times in the reactor in the order of 10-15 days. Given the difference between the hydraulic retention times adopted in the two phases, it is necessary for the methane production reactor to have larger dimensions than the one for hydrogen production. The methane production reactor, 14, is also preferably of the CSTR type, is gas tight to allow anaerobic reaction conditions, and is equipped with measurement sensors and sampling ports analogous to those present in the hydrogen production reactor 13. Passages between the reactors 13 and 14 and discharge of the latter also take place through gas-tight ducts and pumps, to prevent air from entering the reactor. The reactor 14 is dosed volumetrically, through appropriate regulation of the pumps between the two reactors. The gas produced is conveyed to a gasometer; ports are positioned upstream of the gasometer, along the gas conveying duct, and downstream thereof, for sampling and analysing the gas. The methanogenic phase is implemented with equipment and methods well-known in the sector and does not require further detailed description herein.

Finally, the material discharged from the methane production reactor is dehydrated to a water content of 70-80% and subsequently mixed with bulking material, generally lignocellulosic material (i.e. wood fibres previously reduced in size), in proportion in weight of 60-70% dehydrated digested + 40-30% bulking material, and, finally, aerobically composted for 70-90 days until producing the compost destined for use as soil ameliorant. In Fig. 1 the station for dehydration of the material is represented by the element 15, the station for mixing with the bulking materials by the element 16, and the composting station by the element 17. These phases of dehydration, mixing and composting are implemented with equipment and methods well-known in the sector and do not require further detailed description herein.

The example will be further illustrated through the following examples.

### EXAMPLE 1

Below is an example of the process of the invention carried out at laboratory scale. 1 kg of mixture composed of 250 g of MSWOF, 200 g of OMW and 550 g of AS is produced. The water content of this mixture is such that the volume of 1 I corresponds approximately to 1 kg.

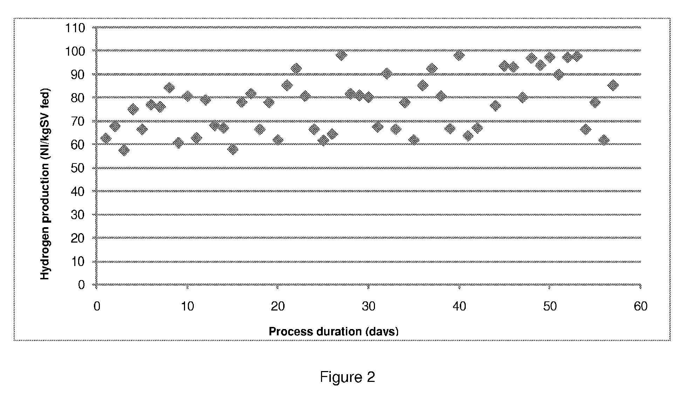

Preparation of said mixture takes place by first subjecting the MSWOF fraction (coming from separate waste collection or mechanical sorting) to a grinding process with blade grinder which reduces the size to around 5 cm. The MSWOF fraction, thus treated, is then sent to a heated mixer to which the OMW fraction and the AS fraction are also fed. The mixture thus obtained has a total solids content (ST) of approximately 6% and a volatile solids content of 91% of the total solids present; consequently 1 kg of mixture contains approximately 60 g of total solids and approximately 58 g of volatile solids. The mixture is homogenised and transferred through a pump to a continuous stirred-tank reactor (CSTR) with a useful volume of 1 litre. The reactor, equipped with sensors to detect pH and temperature, as well as ports to take liquid and gaseous samples to check the trend of the biological process, is heated to 39 °C. Each day a volume of half the reactor volume (approximately 0.5 I) is extracted, so as to set an average hydraulic retention time of the mixture in the reactor of 2 days; the volume of mixture extracted is replaced by the same volume of fresh mixture. The quantity of gas produced each day is measured volumetrically and its composition is analysed using gas chromatography, in order to determine the hydrogen content. Hydrogen production, monitored for almost 60 days, is shown in the graph of Fig. 2, expressed as normal litres of hydrogen produced per kg of volatile solids fed; as can be observed in the figure, the average hydrogen production in these experimental conditions is equal to about 70 NI of hydrogen per kg of volatile solids fed, i.e. approximately 4 NI H₂ per kg of mixture fed. The average percentage of hydrogen in the gas produced is, in these operating conditions, 36% v/v, the rest being essentially CO₂. With regard to the efficiency of the process in terms of biological stabilisation of the mixture of biodegradable waste fed, the average removal of the volatile solids content fed is approximately 30%.

### EXAMPLE 2

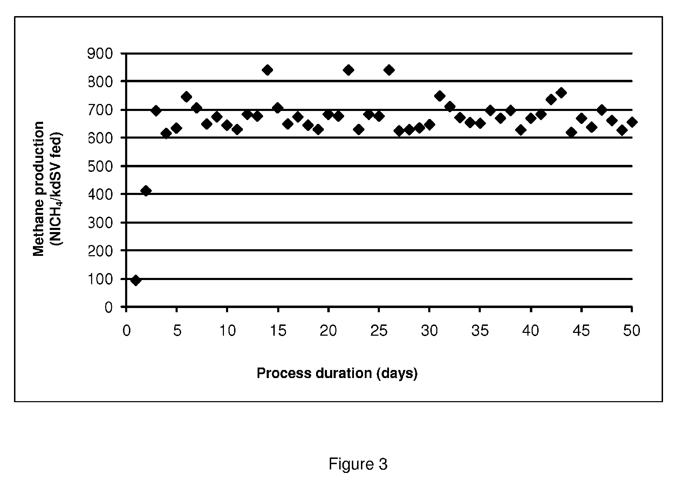

The following example relates to methane production at laboratory scale starting from the mixture extracted from the hydrogen production reactor of Example 1. The mixture extracted from the reactor for the production of hydrogen is characterised by a total solids content of approximately 5.5%, and a volatile solids content of approximately 74% of the total solids present. Consequently, 1 kg of extracted mixture will contain approximately 55 g of total solids and approximately 41 g of volatile solids. As stated in Example 1, the retention time adopted for the hydrogen production reactor is 2 days, and the volume of mixture extracted each day is 0.5 I. This volume is sent directly to a second continuous stirred-tank reactor destined for methane production, maintained constantly at a temperature of 39 °C. In this second reactor a hydraulic retention time of 10 days is adopted; to maintain the mass balance of the two reactors in equilibrium, the useful volume of the second reactor is 5 times the volume of the first reactor, i.e. 5 litres of useful volume. The second reactor too is equipped with ports for taking liquid and gas samples. The quantity of gas produced each day is measured volumetrically and its composition is analysed by gas chromatography, to determine the methane and carbon dioxide content. The average percentage of methane in the gas produced is approximately 70% in volume, the rest being essentially CO₂. The average methane production, monitored for approximately 50 days, is shown in Fig. 3, as normal litres of methane produced per kg of volatile solids fed; as can be seen in the figure, this production is around 700 NI CH₄ per kg of volatile solids fed to the second methanogenic reactor, i.e. approximately 28 NI CH₄ per kg of mixture fed to the second reactor. With regard to the efficiency of the process in terms of biological stabilisation of the mixture of biodegradable waste fed, average removal of the volatile solids content fed to the second methanogenic reactor is approximately 60%, and if the total average removal of volatile solids in the two phases (hydrogen production and methane production) is considered, a total removal of approximately 71% is obtained. By adopting a hydraulic retention time of 10 days for the methanogenic reactor, each day a volume of mixture of 0.5 I is extracted, characterised by a total solids content of approximately 3% and volatile solids content of approximately 59% of the total solids, i.e. 1 kg of mixture extracted from the methanogenic reactor has approximately 28 g of total solids and approximately 16 g of volatile solids.

### EXAMPLE 3

This example relates to compost production at laboratory scale using the mixture extracted from the reactor of Example 2.

The mixture extracted from the methanogenic reactor of Example 2 is sent to a mechanical dehydration station with the purpose of achieving a total solids content of at least 30% in weight. The dehydrated material is then sent to a mixing station. In this station the dehydrated material is mixed with bulking material (wooden chips and straw) in proportions, in weight, of 60-70% of dehydrated material + 40-30% of bulking material. This material is sent to an aerobic composting system where it is kept for approximately 70-90 days. During the composting process the dehydrated material + bulking material mixture sustains a loss in mass of approximately 30% of the wet mass, which takes account of the reduction both of the water content and of the volatile solids content. At the end of the composting phase, the material is extracted and analysed; the result of analysis is shown in Table 1.

**Table 1**

| Parameter | Measure units | Value |
|---|---|---|
| Humidity | % | 65.0 |
| Volatile solids | % ST | 68.8 |
| pH | - | 8.5 |
| Organic C | % ST | 33.8 |
| Humic and fulvic C | % ST | 2.6 |

| Heavy metal content | | |
|---|---|---|
| As | mg/kg | 0.3 |
| Cr | mg/kg | 2.8 |
| Cd | mg/kg | 0.02 |
| Cu | mg/kg | 16.5 |
| Ni | mg/kg | 5.0 |
| Pb | mg/kg | 10.5 |
| Zn | mg/kg | 77.5 |

As can be noted from the results of the experimental examples given, the process of the invention is capable of stably providing high average daily quantities of hydrogen; moreover, the product of the anaerobic hydrogen production station is particularly suitable as input reaction material to feed a methanogenic anaerobic reactor, the solid phase of the product thereof can in turn be used as input component for an aerobic stage of compost production.

## Claims

1. A process for semi-continuous biological hydrogen production through anaerobic digestion at a temperature between 30 and 40 °C of a mixture of waste having a solids content not exceeding 10% in weight, constituted by biodegradable organic fractions of urban waste, both from separate refuse collection and from mechanical sorting, by vegetation waters deriving from olive oil production and by active sludge from urban sewage purification; **characterised in that** the process is conducted with an average residence time of the mixture in the hydrogen production reactor between two and four days, obtained through daily removal of a portion corresponding to approximately 1/n of the volume of mixture in the reactor, where n indicates the number of days of desired average residence time, and feeding an equivalent volume of analogous fresh mixture into the reactor.

2. A process according to claim 1, wherein said average residence time is two days, obtained through daily removal of a portion corresponding to approximately half the volume of mixture in the reactor.

3. A process according to claim 1, wherein said mixture of waste is constituted by between 5 and 25% in weight of vegetation waters from olive oil production, between 45 and 70% in weight of active sludge from urban sewage purification and between 18 and 35% in weight of putrescible organic fractions of urban waste.

4. A process according to claim 3, wherein said mixture of waste is constituted by between 18 and 22% in weight of vegetation waters from olive oil production, between 50 and 60% in weight of active sludge from urban sewage purification and between 22 and 30% in weight of putrescible organic fractions of urban waste.

5. A process according to claim 4, wherein said mixture of waste is constituted by 20% in weight of vegetation waters from olive oil production, 55% in weight of active sludge from urban sewage purification and 25% in weight of putrescible organic fractions of urban waste.

6. A process according to claim 1, wherein said temperature is between 35 and 40 °C.

7. A process according to claim 6, wherein said temperature is approximately 39 °C.

8. A process according to claim 1, further comprising an operation to feed the portion of reaction mixture extracted from said hydrogen production reactor to an anaerobic reactor for methane production, said operation being conducted with an average residence time of said portion of reaction mixture in the methane production reactor approximately between 10 and 15 days at a temperature between 35 and 40 °C.

9. A process according to claim 8, further comprising the operations of dehydrating, mixing with a bulking material, and sending to a composting station, the reacted mixture extracted from the methane production reactor.

10. Anaerobic hydrogen production reactor (13) of the continuous stirred-tank type, equipped with at least one inlet port and at least one outlet port, both gas-tight, with instruments to control pH and temperature of the reaction mixture present in the reactor, and with gas-tight ports to take samples of gas or reaction mixture.

11. Plant for the combined production of hydrogen and methane, constituted by the reactor of claim 10 and by a second gas-tight reactor (14) for methane production, connected in series by means of gas-tight ports and feed systems, so that an outlet port of the hydrogen production reactor (13) is connected to an inlet port of the second reactor (14).

12. A system according to claim 11, wherein said second reactor (14) is of the continuous stirred-tank type and is equipped with instruments to control pH and temperature of the reaction mixture present in the reactor, and with gas-tight ports to take samples of gas or reaction mixture.
